(19) **Europäisches Patentamt**
**European Patent Office**
**Office européen des brevets**

(11) **EP 1 281 752 B1**

(12) **EUROPEAN PATENT SPECIFICATION**

(45) Date of publication and mention
of the grant of the patent:
**20.10.2010 Bulletin 2010/42**

(51) Int Cl.:
*C12N 1/00* (2006.01)      *A23L 1/28* (2006.01)
*A61K 35/66* (2006.01)     *F26B 17/10* (2006.01)

(21) Application number: **01926079.3**

(22) Date of filing: **27.04.2001**

(86) International application number:
**PCT/JP2001/003754**

(87) International publication number:
**WO 2001/083704 (08.11.2001 Gazette 2001/45)**

(54) **METHOD FOR PREPARING SPRAY-DRIED MICROBIAL CELLS**

VERFAHREN ZUR HERSTELLUNG VON SPRÜH-GETROCKNETEN, MIKROBIELLEN ZELLEN

PROCEDE DE FABRICATION DE CELLULES MICROBIENNES SECHEES PAR PULVERISATION

(84) Designated Contracting States:
**AT BE CH CY DE DK ES FI FR GB GR IE IT LI LU
MC NL PT SE TR**

(30) Priority: **02.05.2000   JP 2000133307
07.11.2000   JP 2000339267**

(43) Date of publication of application:
**05.02.2003   Bulletin 2003/06**

(73) Proprietor: **Biofermin Pharmaceutical Co., Ltd.
Kobe-shi,
Hyogo 653-0011 (JP)**

(72) Inventors:
 • **Mizuguchi, Taiji**
   **c/o Biofermin Pharma. Co., Ltd**
   **Kobe-shi**
   **Hyogo 651-2242 (JP)**
 • **Arai, Teruhiko**
   **c/o Biofermin Pharma. Co., Ltd**
   **Kobe-shi**
   **Hyogo 651-2242 (JP)**
 • **Hirayama, Kouichi**
   **c/o Biofermin Pharma. Co., Ltd.**
   **Kobe-shi**
   **Hyogo 651-2242 (JP)**

(74) Representative: **Teipel, Stephan et al
Lederer & Keller
Patentanwälte
Prinzregentenstrasse 16
80538 München (DE)**

(56) References cited:
   **EP-A1- 0 818 529      EP-A2- 0 544 289
   EP-A2- 0 544 289       WO-A-01/44440
   WO-A-98/00035          WO-A-99/02170
   JP-A- 9 048 686        US-A- 5 845 846**

 • **DATABASE BIOSIS [Online] BIOSCIENCES
   INFORMATION SERVICE, PHILADELPHIA, PA,
   US; 1983, OHGKE H ET AL: "A METHOD FOR
   PRODUCTION OF BACTERIA LABELED TALC
   DUST FOR USE IN EXPERIMENTAL AIR
   HYGIENE" XP002445370 Database accession no.
   PREV198376083847 & ZENTRALBLATT FUER
   BAKTERIOLOGIE MIKROBIOLOGIE UND
   HYGIENE ABT 1 ORIGINALE B HYGIENE
   UMWELTHYGIENE KRANKENHAUSHYGIENE
   ARBEITSHYGIENE PRAEVENTIVE MEDIZIN, vol.
   177, no. 3-4, 1983, pages 251-256, ISSN:
   0174-3015**
 • **DATABASE MEDLINE [Online] US NATIONAL
   LIBRARY OF MEDICINE (NLM), BETHESDA, MD,
   US; September 1993 (1993-09), JACKSON M M:
   "A tuberculosis outbreak from a patient's
   draining lesion: how did it happen?"
   XP002445371 Database accession no.
   NLM8212100 & TODAY'S OR NURSE 1993 SEP-
   OCT, vol. 15, no. 5, September 1993 (1993-09),
   pages 29-33, ISSN: 0194-5181**
 • **VARVAROV B.B. ET AL.: 'Physico-chemical
   properties of a small fraction of bakery yeast at
   the moment of drying' INDUSTRIES OF BAKERY
   AND CONFECTIONERY no. 8, 1984, pages 40 - 41,
   XP002941567**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical Field

The present invention relates to a method for the manufacture of a dried microorganism cell product using spray drying.

Background of the Invention

With regard to a drying method in the manufacturing step of pharmaceutical and food, freeze-drying or vacuum freeze-drying has been so much used. Freeze-drying is a method where drying is carried out using liquid nitrogen or the like at the temperature of as low as from about -20°C to about -160°C and vacuum freeze-drying is a method where drying is carried out at about 35°C or lower under the pressure of from about 50 to about 400 hPa.

However, when freeze-drying or vacuum freeze-drying is adopted as a drying method in the manufacturing step for microorganism cells of lactic acid bacteria, yeast, etc. or for a composition such as pharmaceutical or food containing the same, there is a disadvantage that the microorganism cells are killed or damaged during the drying. That is because long time is needed until reaching the freezing temperature in freeze-drying or vacuum freeze-drying and the microorganism is damaged by metabolites of the microorganism produced during that period or by a freezing process. Especially in the case of vacuum freeze-drying, the drying speed is very slow and, if water in the pharmaceutical or food is to be completely removed, the drying time becomes far longer whereby the above-mentioned problem becomes more significant.

Moreover, in carrying out freeze-drying or vacuum freeze-drying, equipment of a very big scale is necessary and much cost is required for investment of the equipment.

Further, when freeze-drying or vacuum freeze-drying is adopted as a drying method, it is difficult to manufacture by means of continuous steps and, therefore, manufacturing steps become troublesome. Furthermore, after freeze-drying or vacuum freeze-drying, additional steps such as grinding and homogenization are necessary whereby manufacturing steps become many and extinction of the microorganism during the steps becomes a problem.

Problems as such have been pointed out for freeze-drying or vacuum freeze-drying.

On the other hand, with regard to a drying method, spray-drying has been known besides freeze-drying or vacuum freeze-drying. Spray-drying is a method where liquid is made into liquid droplets by a device for preparing fine particles and the liquid droplets are contacted with dry wind of a relatively high temperature to dry by evaporation of moisture.

However, in the conventional spray-drying, temperature of the wind for drying is high and microorganism is killed by the heat of the drying wind whereby that has been said to be unsuitable for the manufacture of pharmaceutical or food containing living cells. When temperature of the drying wind is made low for solving such a problem, it is not possible to give a well-dried microorganism cell product whereby there are problems that both yield and stability decrease.

EP-A1-0818529 discloses a process for spray drying in which a composition containing microorganisms is first prepared and then reduced to a powder by spray drying in a spray dryer with an inlet air temperature of 200-400 °C and an outlet air temperature of 40-90 °C and a residence time in the dryer selected so as to ensure survival of >= 1 % of the microorganisms after drying.

An enteral dietary composition comprising two or more lactic acid bacteria which can be live, freeze-dried or heat-killed is known from WO 98/00035.

WO 99/02170 discloses the use of lactobacilli in the preparation of an enteral nutritional composition wherein the lactobacilli can be freeze-dried or spray-dried.

Freeze-drying of suspensions of Micrococcus luteus with talc yielding a wide range of particle diameters is disclosed in Zentralblatt für Bakteriologie, Mikrobiologie und Hygiene, Abt. 1, Originale B, Hygiene Umwelthygiene Krankenhaushygiene Arbeitshygiene Präventive Medizin, Vol. 177, No. 3-4, pages 251-256.

JP 09-048686 discloses a fermentation promoting deodorant obtained by adsorbing a fermentation promoting microorganism on a deodorant and making it into fine particles having 0.5-8.0 $\mu$m average particle diameter.

Disclosure of the Invention

An object of the present invention is to provide a method for drying the liquid of microorganism cells where, in a manufacturing step of a dried microorganism cell product or of a composition such as pharmaceutical or food containing the same, extinction or damage of microorganism cells is suppressed as much as possible and survival rate is able to be highly retained.

Still another object of the present invention is to provide a method for the manufacture of a microorganism cell composition where the manufacturing steps can be greatly simplified.

The present inventors have carried out intensive investigations for achieving the above-mentioned objects and obtained the finding that, when spray-dried liquid droplets of a single micron size are dried by drying wind, problems in the conventional spray-drying can be solved.

Thus, sprayed liquid droplets formed by the conventional spray-drying devices are about 10-40 $\mu$m while, when they are in sprayed liquid droplets of as small as a single micron size, surface area per unit weight of the sprayed liquid droplets becomes bigger whereby contact with drying wind proceeds very efficiently. Accordingly, when drying is carried out by drying wind of the temperature which is in the same degree as that in the conventional spray-drying, the time required for drying the microorganism cell liquid becomes shorter and extinction or damage of the microorganism cells can be suppressed as much as possible. Further, even when the temperature of drying wind is made lower than the temperature of drying wind in the conventional spray-drying, a sufficient drying is possible without deteriorating the yield or stability and, furthermore, since the temperature of drying wind is low, extinction or damage of the microorganism cells due to heat of drying wind can be suppressed as much as possible.

The present inventors have further found that the dried microorganism cell product obtained by drying of sprayed liquid droplets in a single micron size by drying wind have a small particle size and contain particles of a single micron size. A dried microorganism cell product having a small particle size has advantages that the pressure to be applied to the microorganism cells upon compressive molding, etc. is small and static electricity is hardly generated whereupon it is easily made into pharmaceutical or food in a preparation form such as tablets.

Thus, the present invention relates to:

(1) A method for the manufacture of a dried microorganism cell product having a value of the particle size of 1-10 $\mu$m by rounding the first decimal factor, **characterized in that**, sprayed liquid droplets having a value of the particle size of 1-10 $\mu$m by rounding the first decimal factor are dried with drying wind;

(2) The method for the manufacture of the dried microorganism cell product mentioned in the above (1), wherein viable cell count in the dried microorganism cell product is from $1.0 \times 10^5$ to $2.0 \times 10^{12}$ CFU/g;

(3) The method for the manufacture of the dried microorganism cell product mentioned in the above (1) or (2), wherein the microorganism is bifido bacteria, lactobacillus, lactococci, sporogenous lactic acid bacteria, saccharification bacteria, yeasts, koji molds, actinomycetes, *Bacillus toyoi, B. licheniformis*, butyric acid bacteria, acetic acid bacteria or amino acid fermentation bacteria;

(4) The method for the manufacture of the dried microorganism cell product mentioned in the above (1) to (3), wherein the microorganism is bifido bacteria;

(5) The method for the manufacture of the dried microorganism cell product mentioned in the above (1) to (4), wherein the dried microorganism cell product is further coated or masked;

(6) The method for the manufacture of the dried microorganism cell product mentioned in the above (1) to (5), wherein the sprayed liquid droplets having a value of the particle size of 1-10 $\mu$m by rounding the first decimal factor are formed by a four-passage nozzle;

(7) The method for the manufacture of the dried microorganism cell product mentioned in the above (1) to (6), wherein the temperature of drying wind is 5-150°C at the inlet of a drying chamber;

(8) The method for the manufacture of the dried microorganism cell product mentioned in the above (1) to (7), wherein a liquid coating agent or masking agent is sprayed together with a microorganism cell liquid to form sprayed liquid droplets and the said sprayed liquid droplets are dried with drying wind;

(9) The method for the manufacture of the dried microorganism cell product mentioned in the above (8), wherein the microorganism cell liquid is supplied from one liquid passage in a four-passage nozzle having two liquid passages and two gas passages, a liquid coating agent or masking agent is supplied from another liquid passage, compressed gas is supplied from the two gas passages and the resulting sprayed liquid droplets are dried by a drying wind;

(10) The method mentioned in the above (1) to (9), **characterized in that**, a step where a fluidized layer granulation using powder and binder and a drying step where dried microorganism cell product having a value of the particle size of 1-10 $\mu$m by rounding the first decimal factor is obtained by spray-drying are carried out in the same container.

Brief Description of the Drawings

Fig. 1 shows an internal structure of a nozzle edge part in a spray-drying device having a four-passage nozzle.

Fig. 2 shows a rough sketch of a spray-drying device having a four-passage nozzle.

Fig. 3 shows changes in viable cell count during the acid-resistance test of a dried microorganism cell product subjected to an enteric coating.

Fig. 4 shows a rough sketch of a device which is **characterized in that** a step where a fluidized layer granulation using a powder and a binder and a drying step where dried microorganism cell product of a single micron size is obtained by spray-drying are carried out in the same container in accordance with the present invention.

Explanation of symbols:

| | |
|---|---|
| 1, 2 | gas passage wherefrom compressed gas is supplied |
| 3, 4 | liquid passage wherefrom liquid containing a substance to be dried is supplied |
| 5 | fluidized surface of fluid |
| 6 | focus of collision |
| 7 | sprayed liquid droplet |
| 21 | microorganism cell liquid, coating agent or masking agent |
| 22 | passage for liquid |
| 23 | passage for gas |
| 24 | inlet for compressed air |
| 25 | inlet for drying wind |
| 26 | inlet thermometer |
| 27 | device for preparing fine particles |
| 28 | four-passage nozzle |
| 29 | outlet thermometer |
| 30 | pressure gage for inner area of drying chamber |
| 31 | drying chamber |
| 32 | outlet for dried microorganism cell product |
| 33 | rectification device |
| 41 | main body of device |
| 42 | fluidized bed |
| 43 | bug filter |
| 44 | microorganism cell liquid |
| 45 | binder |
| 46 | nozzle for spraying of microorganism cell liquid |
| 47 | nozzle for spraying of binder |
| 48 | powder |
| 49 | gas for fluidization |

Best Mode for Carrying Out the Invention

Dried microorganism cell product usually means each dried microorganism cell or aggregate of dried microorganism cell product.

The method of the present invention provides a dried microorganism cell product having a particle size of a single micron size

Here, the term "single micron" means a value of 1-10 $\mu$m when the first decimal place is rounded.

There is no particular limitation for the microorganism used in the present invention and its examples are bifidobacteria such as *Bifidobacterium bifidum, B. longum, B. breve, B. adrecentis, B. infantis, B. pseudolongum* and *B. thermophilum*; lactobacillus such as *Lactobacillus acidophilus, L. casei, L. gasseri, L. plantalum, L. delbrueckii* subsp. *bulgaricus* and *L. delbrueckii* subsp. *lactis;* lactococci such as *Leuconostoc mecenteroides, Streptococcus (Enterococcus) faecalis, Streptococcus (Enterococcus) faecium, Streptococcus (Enterococcus) hirae, Lactococcus lactis* and *Streptococcus thermophilus*; sporogenous lactic acid bacteria such as *Bacillus coagulans*; saccharification bacteria such as *Bacillus subtilis, Bacillus mesentericus* and *Bacillus polyfermenticus*; yeasts such as *Saccharomyces cerevisiae* and *Candida;* koji molds such as *Aspergillus oryzae, A. niger* and *A. sojae*; actinomycetes such as *Streptomyces*; butyric acid bacteria such as *Bacillus toyoi, B. licheniformis* and *Clostridium butyricum*; acetic acid bacteria such as *Acetobacter*; amino acid fermentation bacteria such as *Corynebacterium*; and other useful microorganisms.

The above-mentioned microorganism cells can be prepared by incubation under the known conditions or the conditions similar thereto. For example, in the case of lactic acid bacteria, one or more of the above-mentioned lactic acid bacteria is/are incubated in a liquid medium containing glucose, yeast extract, peptone, etc. usually at about 25-45°C for about 4-24 hours and the cells are collected from the incubated liquid and washed to give the wet cells.

The microorganism cells may be a processed product of the above-mentioned microorganism cells. Examples of the process are extraction with an appropriate solvent, etc. Specific examples are yeast extract which is an extract of yeast with hot water, etc.

In the method for the manufacture of a dried microorganism cell product in accordance with the present invention, the above-mentioned microorganism cells are firstly dissolved in a solvent to prepare a microorganism cell liquid. With regard to the solvent, although that which has been publicly known in the art may be used, water is preferred. If desired, ethyl alcohol or the like may be added thereto. When ethyl alcohol or the like is added, ethyl alcohol or the like is firstly vaporized and then water is vaporized whereby a stepwise drying is possible.

The microorganism cell liquid may be a suspension. With regard to the solvent, known one may be used as mentioned above although water or aqueous solution where ethyl alcohol or the like is added to water is preferred. In the preparation of a suspension, a suspending agent may be used. With regard to a suspending agent, known ones such as sodium alginate or methyl cellulose may be used.

A protective agent may be added to the above microorganism cell liquid.

The protective agent may be that which has been commonly used in the art and its examples are vitamins such as ascorbic acid; amino acids such as glutamine, glutamic acid, L-cysteine, glycine, phenylalanine, serine or threonine; saccharides or sugar alcohols such as glucose, fructose, sucrose, maltose, mannitol or maltitol; polysaccharides such as oligosaccharide, cyclodextrin or dextrin; fats such as higher fatty acids obtained from rapeseed, soybean, peanut, etc.; proteins such as those obtained from cow's milk, soybean, etc. and degraded proteins such as peptide; inorganic salts such as magnesium sulfate; and others such as sucrose fatty acid ester, malic acid, nucleic acids, yeast extract, skim milk, peptone, gelatin, tannin, etc.

With regard to the protective agent, one of the above-mentioned substances may be used solely or any two or more thereof may be used jointly. Incidentally, it is preferred that the said protective agent is added in an amount of about 0.1-5.0-fold or, preferably, about 0.5-3.0-fold of the weight of the wet microorganism cells.

Additives which have been commonly used in the art such as excipient, binder, disintegrating agent, antistatic agent, etc. may be further added to the above microorganism cell liquid in a common compounding ratio.

With regard to the excipient, there may be used saccharides such as lactose, granulated sugar and corn starch; sugar alcohols such as mannitol; and cellulose such as natural cellulose, crystalline cellulose, etc. and cellulose derivatives such as hydroxycellulose. Examples of the crystalline cellulose are water-insoluble cellulose in white powder prepared by subjecting natural cellulose to, for example, an acidic treatment so that network molecular structure is made fine to some extent, etc. A mixture of crystalline cellulose with carmellose or carmellose sodium may be also exemplified.

Examples of the binder are hydroxypropyl cellulose, hydroxypropyl methylcellulose, methylcellulose, dextrin and α-starch.

Examples of the disintegrating agent are carmellose calcium, cross-linked product (AcDiSol) of carmellose, carmellose sodium, etc., cross-linked product (Plasdone) of polyvinylpyrrolidone, etc., corn starch and lowly substituted hydroxypropyl cellulose.

Examples of the antistatic agent are Syloid, talc and Aerosil.

Then the above-mentioned microorganism cell liquid is subjected to a spray-drying in the method for the manufacture of a dried microorganism cell product according to the present invention. With regard to a spray-drying device used for the said spray-drying, a spray-drying device equipped with a device giving fine particles being able to form sprayed liquid droplets of a single micron size is preferred. This is because, when sprayed liquid droplets having very small particle size are prepared, surface area per unit weight of the sprayed liquid droplets becomes bigger, contact with drying wind is done efficiently whereby extinction or damage of the microorganism by heat of the drying wind can be suppressed as much as possible.

Here, sprayed liquid droplets of a single micron size mean that the particle size of a sprayed liquid droplet is 1-10 μm when its first decimal place is rounded off.

A specific example of the spray-drying device is a spray-drying device where a device for preparing fine particles is a two-passage nozzle or a four-passage nozzle utilizing the force of rotary atomizer (rotary disk), compressing nozzle or compressed gas.

In the present invention, any of the above-mentioned spray-drying devices may be used regardless of the type but, in view of the fact that fine sprayed liquid droplets are able to be prepared, the use of a spray-drying device having a four-passage nozzle is preferred. The said spray-drying device may be that which has been known *per se.*

Structure of the four-passage nozzle in a spray-drying device having a four-passage nozzle is that, when a gas passage and a liquid passage comprise a system, they are symmetrically installed at the nozzle edge in two systems and a slope which is a fluidized surface of fluid is installed at a nozzle edge.

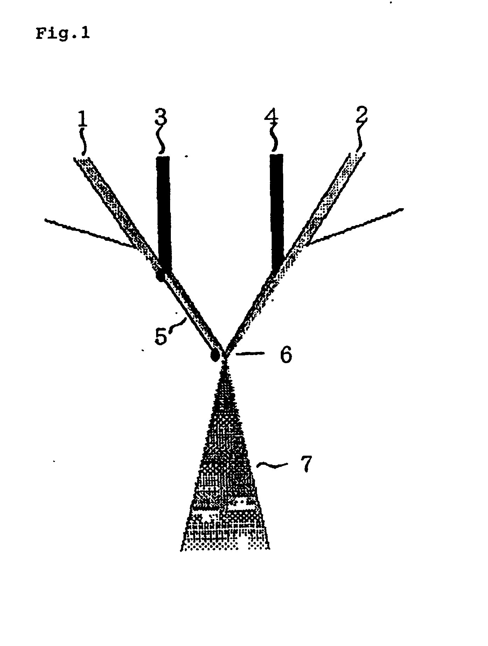

An embodiment of the structure of the four-passage nozzle will be illustrated in more detail by referring to Fig. 1. In a nozzle edge of the four-passage nozzle, the microorganism cell liquid coming out from the liquid passage 3 or 4 as if sprung out therefrom is thinly extended on a fluidized surface of fluid 5 by a high-speed gas flow coming out from the gas passage 1 or 2 and the extended liquid is made into fine particles by shock wave generated at the focus of collision 6 at the front end of the nozzle edge whereupon sprayed liquid droplets 7 of a single micron size are formed.

As such, the preferred four-passage nozzle is that which is in an externally mixing system where compressed gas and liquid are focused to a point from both sides toward the focus of collision of the front end of the nozzle edge.

This is because, in such a system, there is no clogging of the nozzle whereby it is possible to spray for a long period.

With regard to the compressed gas, it is possible, for example, to use air, carbon dioxide gas, nitrogen gas, argon gas and other inert gas. Especially when a thing which is apt to be oxidized is spray-dried, it is preferred to use inert gas such as nitrogen gas or argon gas.

Pressure of the compressed gas is about 1-15 kg weight/cm$^2$ or, preferably, about 3-8 kg weight/cm$^2$.

Amount of the gas at the nozzle per 1 mm of the nozzle edge is about 1-100 L/minute, preferably about 5-50 L/minute or, more preferably, about 10-20 L/minute.

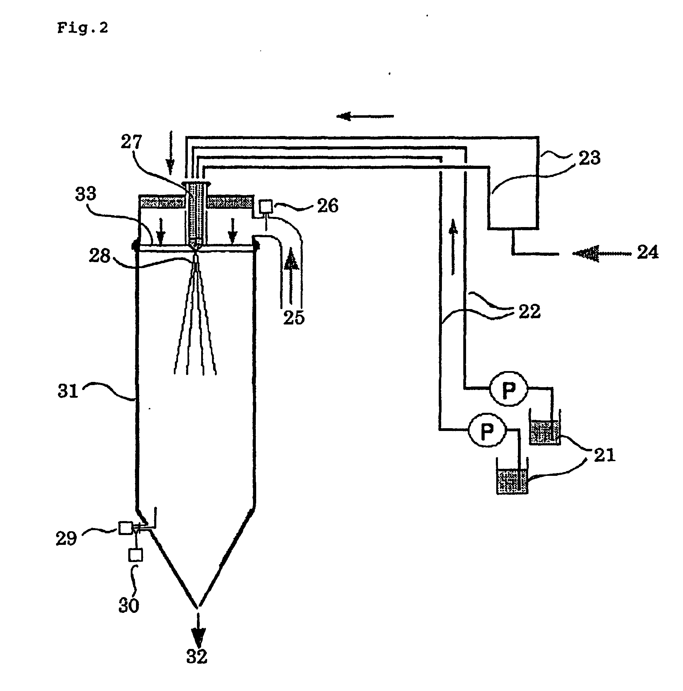

An embodiment of the spray-drying device having a four-passage nozzle is shown in Fig. 2. When an embodiment of the spray-drying according to the present invention is illustrated using Fig. 2, the microorganism cell liquid 21 is sprayed from the four-passage nozzle 28 as illustrated hereinabove by referring to Fig. 1. In a drying chamber 31, the said spray-dried liquid droplets are contacted with drying wind sent via a rectification device 33 from a drying wind inlet 25 whereupon moisture is evaporated to give a dried microorganism cell product.

Temperature of the drying wind at the inlet of the drying chamber (hereinafter, referred to as "inlet temperature") is about 2-400°C, preferably about 5-250°C or, more preferably, 5-150°C. Even when the inlet temperature is as high as about 200-400°C, temperature of the thing to be dried does not become so high due to heat of vaporization of water and, further, extinction and damage of viable cells can be suppressed as much as possible when residence time in the drying chamber is made short.

Temperature of the drying wind at the outlet of the drying chamber (hereinafter, referred to as "outlet temperature") is about 0-120°C, preferably about 5-90°C or, more preferably, about 5-70°C.

In the spray-drying device as shown in Fig. 2, the inlet temperature is the temperature measured by a thermometer at the inlet (26) while the outlet temperature is the temperature measured by a thermometer at the outlet (29).

The drying chamber may be vacuated. As a result of vacuation, there is an advantage that a drying efficiency is improved and drying time becomes shorter. Although the pressure of the drying chamber after vacuation varies depending upon the size of the sprayed liquid droplets, etc. whereby it is not generally definitive, it is made, for example, into the pressure of about a low vacuum region or, to be more specific, from about $1.0 \times 10^3$ to $7.0 \times 10^3$ Pa.

In case a spray-drying device having a four-passage nozzle is used, there are two liquid passages in a nozzle as mentioned above and, therefore, when two different microorganism cell liquids or a microorganism cell liquid and another solution or suspension are sprayed from different liquid passages at the same time, it is possible to manufacture a dried microorganism cell product wherein they are mixed. For example, when microorganism liquids of two different kinds of microorganisms are sprayed at the same time, a dried microorganism cell product containing the said two kinds of microorganisms is obtained. When a microorganism cell liquid and an edible composition are sprayed at the same time, a dried microorganism cell product containing the microorganism cells and the edible composition is obtained.

Examples of the edible composition used in the present invention are a liquid composition of milk, meat, fish, fruit or vegetable. Two or more kinds of them may be mixed therein.

It is preferred that such an edible composition is concentrated to a water content of less than about 70% by weight before the spray-drying where water remained to an extent that the fluidity is not lost.

Examples of the above-mentioned liquid composition of fruit or vegetable are a solution or a suspension where seed, root, tuber, stem, leaf, flower or fruit which is finely ground with or without heating is dissolved or suspended in an appropriate solvent.

Preferred examples of fruit or vegetable are leaves of leek, Welsh onion, asparagus, fennel, cabbage, etc.; stems of rhubarb, broccoli, etc.; seeds of cocoa, pea, soybean or cereals; roots of carrot, onion, *hatsukadaikon* (a kind of Japanese radish), celery, beet, etc.; tubers of casaba, potato, etc.; fruit such as tomato, courgette, eggplant, banana, apple, apricot, melon, watermelon, pear, plum, peach, cherry, kiwi, sea buckthorn berry, medlar, mirabelle cherry, etc.; and mushroom; etc.

Examples of a liquid composition of milk, meat or fish are a solution or a suspension where milk, egg or meat or fish which is finely ground with or without heating is dissolved or suspended in an appropriate solvent; protein obtained therefrom or its hydrolyzed protein; etc.

In a spray-drying device having a four-passage nozzle, when a microorganism cell liquid is sprayed from one liquid passage while a liquid coating agent of masking agent is sprayed from another liquid passage in the two liquid passages of the nozzle, the microorganism cells can be coated or masked whereupon a coated or masked dry microorganism cell product can be prepared.

As a result of coating of the microorganism cells, it is possible to adjust the time for disintegration or to adjust so as to act in specific organs such as intestine. It is also possible to attempt an improvement of appearance or enhancement of quality. Further, in the case of the pharmaceutical or food containing two or more ingredients and when denaturation takes place by contacting the said ingredients, it is now possible to suppress the denaturation by coating one of or both ingredients.

It is furthermore possible to suppress bitter taste or acidic taste by masking the microorganism cells. Especially, pharmaceutical or food containing high concentrations of lactic acid bacteria or the like has a unique taste and there is an advantage that, when all or a part of the surface of the microorganism cells is coated or masked, such a unique taste can be reduced.

With regard to the coating agent used in the present invention, there may be used known materials having a coating ability such as hydroxypropyl cellulose (hereinafter, abbreviated as HPC), starch paste, silicone, solution of sugar such as starch syrup or various coating materials.

Examples of the material for enteric coating are hydroxypropyl methylcellulose phthalate (HPMCP), hydroxypropyl methylcellulose acetate succinate (HPMCAS), cellulose acetate phthalate (CAP), carboxymethyl ethylcellulose (CMEC), methacrylic acid-ethyl acrylate copolymer and methacrylic acid-methyl methacrylate copolymer. Examples of other coating material are ethylcellulose, hydroxypropyl methylcellulose, cellulose acetate phthalate and polyvinylacetal diethylaminoacetate.

Examples of the masking agent used in the present invention are aminoacryl methacrylate copolymer E, aminoacryl methacrylate copolymer RS, methacrylic acid copolymer LD and HPC.

When such a coating agent or a masking agent is liquid, the said agent may be used as it is. Alternatively, it may be diluted with a diluent such as water. When the said agent is solid, it is dissolved or suspended in a solvent such as water and is used.

It is also possible that, in place of a coating agent or a masking agent, a wall membrane substance is sprayed from a liquid passage of the four-passage nozzle whereupon microcapsules can be formed.

In that case, it is recommended that the microorganism cell liquid is prepared by suspending the microorganism in a solvent such as glycerol, medium-chain triglyceride or corn oil which is inert to the microorganism. This is because, when the solvent for the microorganism cells is water, survival rate of the microorganism cells lowers due to the products as a result of a brisk metabolism of the microorganism.

Examples of the wall membrane substance are a cellulose substance such as ethylcellulose; a high-molecular substance such as polylactic acid, polystyrene, polybutadiene and styrene-methyl acrylate copolymer; and hydrogenated oil having a melting point of not lower than body temperature such as hydrogenated palm oil.

When such a wall membrane substance is liquid, the said substance may be used as it is. Alternatively, it may be diluted with a diluent such as water. When the said substance is solid, it is dissolved or suspended in a solvent such as water and is used.

With regard to a method for the manufacture of a dried microorganism cell product which is coated or masked according to the present invention or a method for the manufacture of microcapsules containing the dried microorganism cell product, there may be exemplified not only the above-mentioned method but also a method where the above-mentioned microorganism cell liquid is previously mixed with a coating agent, a masking agent or a wall membrane substance and the resulting mixed liquid is subjected to spray-drying according to the present invention.

With regard to the dried microorganism cell product obtained by the above-mentioned spray-drying according to the present invention, it is possible to prepare a dried microorganism cell product having a desired particle size by adjusting the gas amount of the compressed gas and the liquid amount of the microorganism cell liquid sent to the nozzle upon checking whether the microorganism cell liquid is a solution or a suspension or checking its concentration and property such as viscosity.

As mentioned already however, the particle size of the dried microorganism cell product is 1-10 $\mu$m by rounding the first decimal factor. This is because, when the particle size is small, static electricity is hardly generated during the step of preparation or pressure to the microorganism cells upon tabletting is small. Incidentally, particle size of the dried microorganism cell product can be easily measured under a microscope.

In order to make the particle size of the resulting dried microorganism cell product small, it is also possible that a treatment which is known *per se* is carried out before the spray-drying. Examples of the known treatment are a treatment where viscosity and surface tension of the microorganism cell liquid are made small and a treatment where the primary particle size is made small or suspending is well conducted when the microorganism cell liquid is a suspension of microorganism cells.

When the particle size of the dried microorganism cell product is made small as mentioned above, there is another advantage that viable cell ratio rises and pharmaceutical or food containing many viable cell count can be provided. Thus, in obtaining a dried microorganism cell product of a single micron size, the sprayed liquid droplets are in a single micron size. When particle size of the sprayed liquid droplets is small, surface area per unit weight of the sprayed liquid droplets becomes large whereupon contact with drying wind takes place efficiently and, even when temperature of the drying wind is relatively low, an efficient drying is possible whereby extinction or damage of the microorganism cells by heat of the drying wind can be suppressed as much as possible. As a result, viable cell ratio rises to give a dried microorganism cell product having many viable cells.

Therefore, in accordance with the spray-drying of the present invention, it is possible to prepare a dried microorganism cell product where the viable cell count is from about $1.0 \times 10^5$ to about $2.0 \times 10^{12}$ CFU/g, preferably from about $1.0 \times 10^{11}$ to about $2.0 \times 10^{12}$ CFU/g, more preferably from about $1.2 \times 10^{11}$ to about $1.5 \times 10^{12}$ CFU/g and, most preferably, from about $1.5 \times 10^{11}$ to about $8.0 \times 10^{11}$ CFU/g. Measurement of viable cell count in the dried microorganism cell product varies depending upon the microorganism and, for example, it can be easily measured by a quantitative determination method for each microorganism mentioned in the "Japanese Pharmaceutical Codex".

In the present invention, it is also possible to combine the above-mentioned spray-drying with a fluidized layer granulation. Thus, it is possible that a device which is characterized that a step where a fluidized layer granulation is carried out using a powder and a binder and a step where a dried microorganism cell product in a single micron size is prepared by spray-drying are carried out in the same container is used and, in the said device, granulation, spray-drying of microorganism cells and mixing are continuously carried out.

Up to now, steps of spray-drying, granulation and mixing have been carried out in different devices for each but, when granulation, spray-drying of microorganism cells and mixing are carried out in the same device as such, there are advantages that the manufacturing steps are simplified and, at the same time, homogeneity of a mixture of dried microorganism cell product and granules is improved and classification hardly takes place.

An embodiment of the said device is a device where there are installed a nozzle for spraying the binder by which a binder can be sprayed from the upside to the downside of the drying chamber and a nozzle for spraying of microorganism cell liquid from the upside to the downside of the drying chamber and there is also installed a fluidized layer at the bottom of the device by which the powder can be kept in a fluidized state when the air in the device is sucked by an exhaust fan. Here, the air comprising the fluidized layer may be that which is dried by a dehumidifier known *per se.*

In the said device, it is possible that two spraying nozzles are installed therein and each of them is used as a nozzle for spraying the binder and a nozzle for spraying the microorganism cell liquid and it is also possible that only one spraying nozzle is installed therein and the said spraying nozzle is used as a nozzle for spraying the binder and also as a nozzle for spraying the microorganism cell liquid.

As hereunder, there will be mentioned a specific embodiment of a method for the manufacture of a microorganism cell composition, **characterized in that**, a step where a fluidized layer granulation using a powder and a binder and a drying step where a dried microorganism cell product of a single micron size is obtained by spray-drying are carried out using the above-mentioned device. In the following embodiment, a step of a fluidized layer granulation is firstly conducted and then a drying step is conducted although those steps may be done at the same time.

Firstly, a fluidized layer granulation is carried out using a powder and a binder. To be specific, powder is aggregated either by (a) blowing compressed gas, preferably inert gas such as nitrogen gas or carbon dioxide gas, therein from the bottom of the device or by (b) sucking the air in the device using an exhaust fan whereby the binder is sprayed by a nozzle for spraying the binder while the powder is kept in a flowing state so that liquid droplets of the binder are contacted with the fluidized powder.

With regard to a nozzle for spraying the binder, that which is known *per se* may be used although it is preferred to use the above-mentioned four-passage nozzle. When a four-passage nozzle is used, sprayed liquid droplets of the binder become smaller whereby particle size of the granules becomes smaller as well. As a result, time required for drying of the granules becomes shorter making the manufacture efficient. Further, when a four-passage nozzle is used, it is possible to prepare the granules having a relatively uniform particle size. As a result, the steps of grinding and particle size selection can be omitted and, in addition, classification hardly takes place upon mixing of the dried microorganism cell product with the granules.

Incidentally, particle size of the granules is preferably about 100-500 μm.

After that, a microorganism cell liquid is sprayed from a nozzle for spraying the microorganism cell liquid to prepare a dried microorganism cell product of a single micron size. At that time, in succession to the above stage, compressed gas which is blown thereinto from the bottom of the device or gas in the device sucked by an exhaust fan (hereinafter, they will be generally called "gas for fluidization") also acts as drying wind and dries the sprayed liquid droplets of the microorganism cell liquid and, at the same time, it also conducts the mixing with the granules which are in a fluidized state by the gas for fluidization.

Not only the case where particles of the granules and particles of the dried microorganism cell product are independent each other and are mixed together but also the case where particles of the power and particles of the dried microorganism cell product form one particle by a binder and also the case where the said particle and particles of granules and/or particles of the dried microorganism cell product are mixed are exemplified as the mode of the resulting mixture. A microorganism cell composition which is **characterized in that** a dried microorganism cell product of a single micron size is adhered to granules comprising powder and binder as such can be easily manufactured.

With regard to a nozzle for spraying the binder, that which is known *per se* may be used although it is preferred to use the above-mentioned four-passage nozzle. This is because, when a four-passage nozzle is used, the advantage of the four-passage nozzle in the case of the above-mentioned spray-drying is able to be achieved in this manufacturing method as well.

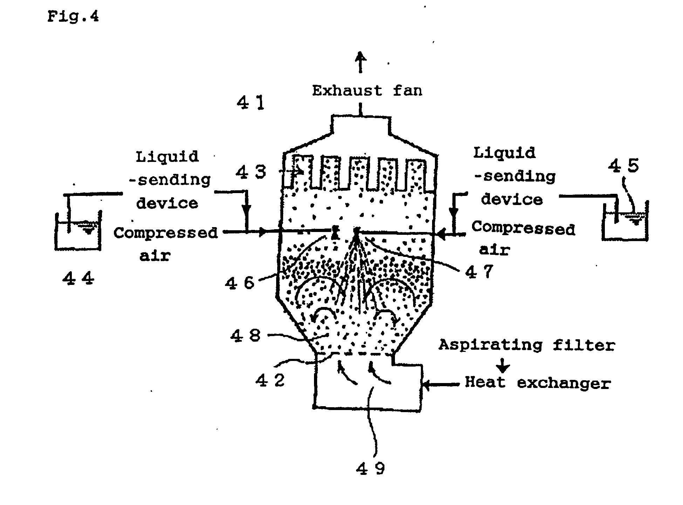

The above-mentioned device and an embodiment of the method for the manufacture of a microorganism cell composition in accordance with the present invention will be illustrated by referring to Fig. 4.

With regard to the fluidized layer granulation, under the state where powder 48 is placed in the main body of the device 41 according to the present invention equipped with a fluidized bed 42, an exhaust fan is driven together with spraying of a binder 45 from a nozzle for spraying the binder 47 and gas for fluidization 49 heated by a heat exchanger is continuously introduced from the side of the lower part of the main body of the device 41. At that time, the gas for fluidization 49 may be previously dried using a dehumidifier (not shown). The introduced gas for fluidization 49 is sucked upward by an exhausting fan. When velocity of the gas for fluidization 49 becomes high, the powder 48 is blown up from the fluidized bed 42 and floated/suspended in the air whereupon the so-called fluidized layer is formed. The liquid droplets of the binder 45 are contacted with the said fluidized powder 48 to aggregate the powder 48 whereby granulation can be carried out.

With regard to spray-drying, when a microorganism cell liquid 44 is sprayed using a nozzle 46 for spraying the microorganism cell liquid, sprayed liquid droplets of the said microorganism cell liquid are dried by the gas for fluidization 49 which is pulled upward from the fluidized bed 42 by an exhausting fan. At that time, it is appropriate to lower, if desired, the temperature of the gas for fluidization 49 than that in the case of fluidizing granulation to an extent of about 2-400°C, preferably about 5-250°C, more preferably about 5-150°C or, still more preferably, about 20-70°C. This is because extinction or damage of the microorganism cells by heating of the gas for fluidization is suppressed as much as possible.

The granules and the dried microorganism cell product manufactured as such are mixed by a gas for fluidization 49 in the device 41.

Although there is no particular limitation for the above powder used for the fluidized layer granulation, that which is able to be administered or taken together with the dried microorganism cell product.

Specific examples thereof are a substance having a pharmacological action other than the dried microorganism cell product, an excipient and other finely-powdered food material. With regard thereto, that which has been known *per se* may be used or a mixture of two or more thereof may be used.

Examples of the said finely-powdered food material are spices such as dried and ground spices and herbs (e.g., cinnamon, ginger, mustard, nutmeg, pepper, saffron, sesame, turmeric, parsley, mint, celery, onion and garlic) and other spices prepared by mixing the above spices and herbs such as curry powder and mixed spice; powdery juice of fruit such as apple, orange, lemon, strawberry, pineapple, grapefruit, grape, melon and lime; powdery beverage such as coffee, green tea, English tea, oolong tea, cocoa and barley tea; powered extract of animal such as beef extract, pork extract, chicken extract, scallop extract, *asari* (short-necked clam) extract, oyster extract, crab extract, shrimp extract and dried bonito extract; powdered extract of plants such as garlic extract, onion extract, celery extract, cabbage extract and trefoil extract; powdery milk product such as skim milk, powdered milk containing fat and fermented milk powder; egg yolk powder and total egg powder; and cereal powder, hydrolyzed protein of animals and plants, table salt, amino acids and seasoning powder of a nucleic acid type.

The binder for the fluidized layer granulation is an additive which enhances the binding force among the particles of the powder or between the dried microorganism cell product and the particles of the powder, and its representative example is a water-soluble high-molecular substance. To be more specific, there may be exemplified hydroxypropyl cellulose, hydroxypropyl methylcellulose and methylcellulose. Besides the above, it is also possible to use polyvinylpyrrolidone, gelatin, polyvinyl alcohol, starch paste liquid or sugar liquid as a binder.

If desired, a compounding agent may be added to the binder. With regard to the compounding agent, that which has been known *per se* may be used and its examples are natural or synthetic surface-active agent such as lecithin, quillaia saponin, sucrose fatty acid ester, glycerol fatty acid ester, polyglycerol fatty acid ester and propylene glycol fatty acid ester; protein such as gelatin, casein, water-soluble peptide, soybean protein, albumin and gluten; etc.

The pharmaceutical or food containing the dried microorganism cell product may contain other ingredients provided that it contains the dried microorganism cell product.

For example, it may contain ingredients having other pharmacological actions or other foods and, if desired, it may contain additives or filling materials having other desired functions.

The dried microorganism cell product prepared by spray-drying as mentioned above can be made into the pharmaceutical or food containing the dried microorganism cell product either as it is in a form of diluted powder or granules or as a form of tablets, pills or troches.

When the pharmaceutical or food containing the dried microorganism cell product is in a form of diluted powder or granules, the dried microorganism cell product obtained by the above spray-drying may be used as it is or may be subjected to a treatment known *per se* being carried out in the art.

When the pharmaceutical or food containing the dried microorganism cell product is in a form of tablets, it is manufactured by tabletting the dried microorganism cell product obtained by the above spray-drying using a tabletting machine. With regard to the tabletting machine, known ones such as a tabletting machine of a rotary type may be used.

It is also possible that the dried microorganism cell product obtained by the above spray-drying according to the present invention is mixed with an additive which is usually powdery or granular and has been commonly used in the art such as a excipients, a binder, a disintegrating agent or an antistatic agent in a usual compounding ratio and the resulting mixture is tabletted by a tabletting machine.

It is further possible that the resulting tablets are subjected to coating or masking such as sugar coating, enteric coating or film coating. With regard to a coating agent or a masking agent, known ones such as the above-mentioned ones may be used. Incidentally, coating or masking may be carried out by a known method or a method similar thereto.

The above-mentioned tablets may be sustained-released tablets. Examples of the sustained-released tablets are gradumet where the dried microorganism cell product obtained by the above spray-drying is dispersed in a plastic lattice which is discharged outside the body as it is; wax matrix where the dried microorganism cell product obtained by the above spray-drying is dispersed in a wax lattice in a spongy shape; resinate where the dried microorganism cell product obtained by the above spray-drying is adsorbed with ion-exchange resin; and spantab comprising a plurality of layers where dissolving and releasing properties are different. Those sustained-released tablets may be manufactured by a known method or by a method similar thereto.

When the pharmaceutical or food containing the dried microorganism cell product is in a form of pills or troches, they may be manufactured by the same manner as in the case of tablets. In the case of troches however, no disintegrating agent is usually added since they are gradually dissolved or disintegrated in the mouth.

The dried microorganism cell product obtained by the above spray-drying may be filled in capsules to prepare a capsule preparation. With regard to a capsule material, known capsule materials such as gelatin may be used. In the case of a soft capsule preparation, there may be used a plasticizer such as glycerol or sorbitol. It is also possible that the dried microorganism cell product obtained by the above spray-drying is subjected to a compressive molding by means of tabletting for example and the molded one is impregnated in fat/oil or the like if necessary, then coated with solid fat/oil and coated with a capsule material. As a result thereof, water does not directly contact with the dried microorganism cell product even when water is permeated inside through the capsule coat whereby damage or extinction of the microorganism cells by water can be prevented.

It is also possible that microcapsule is prepared using polylactic acid or the like according to the conventionally known method.

The pharmaceutical or food containing the dried microorganism cell product may be in a liquid form such as pharmaceutically acceptable solution, suspension, syrup or elixir.

Examples of the diluent used are pure water, vegetable oil and ethanol. Besides the diluent, it is also possible to mix with an additive such as moisturizer, suspending agent, sweetener, flavor, aromatizer and antiseptic.

It is preferred to prepare the suspension in such a manner that the dried microorganism cell product prepared by the above spray-drying is added to a solvent such as water or vegetable oil together with a suspending agent or other desired additive and suspended by a known method so as to make the mixture homogeneous. With regard to the suspending agent, known ones such as gum arabic, carmellose sodium, methylcellulose and sodium alginate may be used.

It is preferred to prepare the syrup preparation in such a manner that the dried microorganism cell product prepared by the above spray-drying is dissolved or suspended in a diluent such as pure water together with sucrose and, if desired, with other saccharide or sweetener. It is also possible to prepare a dry syrup preparation which is used by dissolving or suspending the dried microorganism cell product upon actual use.

It is preferred to prepare the elixir preparation in such a manner that the dried microorganism cell product prepared by the above spray-drying is dissolved in sweet and aromatic ethanol together, if desired, with additives. The amount of ethanol is preferably about 4-40% by weight.

It is also possible that the pharmaceutical containing the dried microorganism cell product is made into parenteral injection preparations such as aqueous injection, non-aqueous injection, aqueous suspension injection or non-aqueous suspension injection.

It is preferred to prepare the injection preparation in such a manner that the dried microorganism cell product prepared by the above spray-drying is dissolved in a solvent together, if desired, with additives followed by filtering or is suspended in a solvent and then charged in a container, the container is tightly sealed and sterilization is carried out.

Examples of the solvent are aqueous solvent such as distilled water for injection, physiological saline and Ringer's solution; and non-aqueous solvent such as propylene glycol, polyethylene glycol, vegetable oil such as olive oil and alcohol such as ethanol.

Examples of the additive are (a) stabilizer such as ethylenediamine; (b) preservative such as p-hydroxybenzoate, benzyl alcohol, chlorobutanol, phenol and cresol; (c) surface-active agent such as polyoxyethylene hydrogenated castor oil and sucrose fatty acid ester or solubilizer such as lecithin and hydrogenated lecithin; (d) isotonizing agent such as sodium chloride, glucose and glycerol; (e) buffer such as phosphate and citrate; and (f) suspending agent such as gum arabic, carmellose sodium, methylcellulose and sodium alginate.

The pharmaceutical or food containing the dried microorganism cell product may also be used for promoting the healthy state of mammals. It may be also used as agricultural chemical or as additive for feed.

For example, lactic acid bacteria have been known as one of the useful enteric bacteria and have been said to be deeply participated in health. Particularly with regard to physiological significance of bifidobacteria, lactococci and lactobacillus, there have been many reports whereby their action of suppressing the malignant microorganisms by production of organic acid such as lactic acid or acetic acid in the intestine, their production of vitamins, their immunological enhancement, etc. have been made clear. Accordingly, there have been developed many products such as pharmaceuticals or foods containing lactic acid bacteria with an object of maintenance and enhancement of health and prevention or therapy of diseases of human being, livestock or pet animals.

In addition, lactic acid bacteria have been said to have deeply participated in the improvement of taste and preservation or to the maintenance of quality during the manufacturing steps of food such as soybean paste, soybean sauce, pickles and *sake* (Japanese rice wine) and have been utilized therefor.

The pharmaceutical containing lactic acid bacteria or bifidobacteria is able to be used for the therapy or prevention of diarrhea, constipation, hypercholesterolemia, hepatic disease, immunosuppressed disease, enteritis (such as gastroenteritis or colitis), etc.; recovery of intestine after disorder by food, pharmaceutical, chemical or physical pharmaceutical or after surgical invasion, chemotherapy or contagious disease; and for the inhibition of absorption of endotoxin and also for the antagonism against the formation of endogenous toxin.

This is because the said pharmaceutical has an action of recovering the intestinal mucous membrane and also adjusting the enzyme potential of intestinal tract when it changes as a result of the state of stress. This is also because it has an action of normalizing the function of intestinal flora, promoting the synthetic, digestive or enzymatic and absorptive steps of vitamins and proteins, preventing the formation of colonies of pathogenic microorganisms and stimulating the immunoreaction.

An example of the use of the food containing the dried microorganism cell product of lactic acid bacteria or bifidobacteria is that the dried microorganism cell product prepared by the above spray-drying is added to milk product such as yogurt or cheese, confectionery or beverage.

It is also possible that the dried microorganism cell product prepared by the above spray-drying *per se* is used as a seasoning. It is further possible that the dried microorganism cell product prepared by the above spray-drying is dissolved or suspended in a solvent such as water or oil and is used as a seasoning. It is furthermore possible that it is mixed with other seasonings.

The dried microorganism cell product of lactic acid bacteria prepared by the above spray-drying is able to be used as a starter for a stable production of food by lactic acid fermentation on an industrial scale.

Powdery lactic acid bacteria starter may be used, for example, for milk products, rye bread and fermented sausage.

The dried microorganism cell product of yeast prepared by the above spray-drying is able to be used in wine industries such as beer, *sake* (Japanese rice wine) and wine utilizing the property that yeast briskly carries out alcohol fermentation. It is also able to be used for fermentation of bread or the like.

Further, since yeast extract which is an autolyzed liquid or extract with hot water of yeast contains a lot of vitamins, organic bases or amino acids and is of a high nutritive value, the dried microorganism cell product of yeast extract prepared by the above spray-drying is able to be used as pharmaceutical such as a vitamin preparation and as a health food.

The dried microorganism cell product of koji mold prepared by the above spray-drying is able to be used for the manufacture of *sake* (Japanese rice wine), *amazake* (a sweet drink made from fermented rice), *shochu* (Japanese distilled spirits), soybean paste, *mirin* (sweet *sake* for seasoning) or soybean sauce. There are many cases where *Aspergillus oryzae* is used although, for the manufacture of alcohol, *Asp. niger* may be sometimes used instead.

In addition, many of koji mold strains produce various kinds of enzymes such as amylase and protease, various kinds of organic bases, vitamins, etc. and, therefore, the dried microorganism cell product of koji mold prepared by the above spray-drying in accordance with the present invention is able to be used as pharmaceutical such as a digestive enzyme preparation and a vitamin preparation or as health food.

Example 1

(Incubation of microorganism cells)

Incubated liquid of *Bifidobacterium bifidum* which was previously grown in a GAM medium (manufactured by Nissui Seiyaku K. K.) was inoculated in 0.1% by weight to the same medium and then incubated at 37°C for 16 hours. This was centrifuged and the resulting precipitate was used as wet microorganism cells.

(Preparation of microorganism suspension)

To an aqueous solution of 0.5M of mannitol, 0.5M of sodium glutamate, 0.1M of arginine, 0.1M of sodium succinate and 0.01M of magnesium sulfate was added dextrin to make 35% by weight and in this dispersing medium prepared as such were suspended the above-prepared wet microorganism cells.

(Measurement of viable cell count in the suspension of the microorganism)

Measurement was carried out by a quantitative determination method for bifidobacteria mentioned under the item of " Bifidobacteria" in the "Japanese Pharmaceutical Codex". Thus, 1 mL of the microorganism suspension was taken and diluted using a diluent prepared as mentioned below by a ten-fold dilution method so as to make the viable cell count 20-200 per mL. This liquid (1 mL) was placed on a Petri dish and 20 mL of agar medium for the test of bifidobacteria kept at 50°C were added thereto followed by quickly mixing to solidify. This was subjected to an anaerobic culture at 37°C for 60 hours and viable cell count in the microorganism suspension was determined from the appeared colony numbers and the diluting ratio.

The agar medium for the test of bifidobacteria was prepared in such a manner that, among the following components, those except equine blood were dissolved by heating, adjusted to pH 7.2, sterilized by heating at 115°C for 20 minutes using a high-pressure steam sterilizer and cooled down to 50°C and equine blood was added thereto.

| | |
|---|---|
| Percolate of bovine liver | 150 mL |
| Peptone prepared from meat | 10 g |
| Peptone made of casein | 5 g |
| Yeast extract | 5 g |
| Meat extract | 3 g |
| Peptone made of soybean | 3 g |
| Glucose | 10 g |
| Potassium monohydrogen phosphate | 1 g |
| Potassium dihydrogen phosphate | 1 g |
| Starch | 0.5 g |
| L-Cysteine hydrochloride | 0.5 g |
| Magnesium chloride | 0.2 g |
| Sodium chloride | 0.01 g |
| Ferrous sulfate | 0.01 g |
| Manganese sulfate | 7 mg |
| Polysolvate 80 | 1 g |
| Agar | 15 g |
| Equine blood | 50 mL |
| Pure water | 790 mL |
| pH: 7.1-7.3 | |

The above-mentioned diluent was prepared in such a manner that the following components were mixed and sterilized by heating at 121°C for 15 minutes using a high-pressure steam sterilizer.

| | |
|---|---|
| Anhydrous sodium monohydrogen phosphate | 6.0 g |
| Potassium dihydrogen phosphate | 4.5 g |
| Polysolvate 80 | 0.5 g |
| L-Cysteine hydrochloride | 0.5 g |
| Agar | 1.0 g |
| Pure water | 1000 mL |
| pH: 6.8-7.2 | |

(Spray-drying)

The microorganism suspension prepared as mentioned above was spray-dried by a spray-drying machine as shown in Fig. 2 where a four-passage nozzle (as mentioned in the Japanese Patent No. 2,797,080) was integrated. Thus, the microorganism suspension was supplied from the two liquid passages at the rate of 8 mL/minute (4 mL/minute/l mm nozzle) while compressed gas was supplied from the two gas passages at the rate of 40 NL/minute (converted value at 20°C) and the resulting sprayed liquid droplets were dried by drying wind to prepare the dried microorganism cell product of the present invention. Incidentally, the drying wind was supplied at the inlet temperature of 100°C and at the rate of 1.0 $m^3$/minute into the drying chamber and its outlet temperature was 67°C.

(Measurement of viable cell count in the dried microorganism cell product)

Viable cell count in the resulting dried microorganism cell product was measured according to a quantitative determination method for bifidobacteria mentioned under the item of "Bifidobacteria" in the "Japanese Pharmaceutical Codex" . Thus, 5 g of the dried microorganism cell product were taken to the same diluent mentioned above which was previously warmed at 37°C whereupon the total volume was made 50 mL. This was well stirred and then warmed at 37°C for 30 minutes to prepare an original sample solution. This was diluted with a diluent liquid by a 10-fold dilution method so as to make the viable cell count 20-200 in 1 mL. This liquid (1 mL) was placed on a Petri dish and 20 mL of agar medium for the test of bifidobacteria kept at 50°C were added thereto followed by quickly mixing to solidify. This was subjected to an anaerobic culture at 37°C for 60 hours and viable cell count in the microorganism suspension was determined from the appeared colony numbers and the diluting ratio.
The same experiments were further carried out where the inlet temperature of the drying wind was made 80°C, 50°C and 50°C successively.

Comparative Example 1

The microorganism suspension prepared by the same manner as in the Example was freeze-dried. The freeze-drying condition was that the shelf temperature was 30°C or lower and the final degree of vacuum was 0.1 Torr (13.3 Pa).
Viable cell count in the powder after drying was measured by the same manner as in Example 1.
Viable cell count and survival rate in the Example and the Comparative Example are shown in Table 1.
Here, the survival rate was calculated by the following formula.

$$\text{Survival rate (\%)} = \{((\text{Viable cell count per gram of dried microorganism cell product}) \times (\text{Solid content of the microorganism suspension}))/(\text{Viable cell count per gram of microorganism suspension})\} \times 100$$

Table 1

| | Inlet Temp (°C) | Exhaust Temp (°C) | Viable Cell Count in Powder (CFU/g) | Survival Rate (%) | Comparison with Comp. Ex.(%) |
|---|---|---|---|---|---|
| Example | 100 | 67 | $1.65 \times 10^{11}$ | 52 | 104 |
| | 80 | 56 | $1.80 \times 10^{11}$ | 55 | 113 |
| | 60 | 44 | $2.28 \times 10^{11}$ | 72 | 143 |
| | 50 | 37 | $2.43 \times 10^{11}$ | 77 | 153 |
| Comp. Ex. | | | $1.59 \times 10^{11}$ | 50 | 100 |
| (Note: Comp. Ex. means Comparative Example) | | | | | |

As shown in Table 1, it has been found that, when the spray-drying in accordance with the present invention is carried out, dried microorganism cell products having a higher survival rate than that by freeze-drying are able to be prepared.

(Particle size of dried microorganism cell product)

The dried microorganism cell product prepared by making the inlet temperature 60°C in Example 1 was measured under a microscope (Digital HD Microscope VH-7000 manufactured by Keyence Corp.). Distribution of the particle size was as follows.

Table 2

| Particle Size | Ratio (%) |
|---|---|
| 1-5 $\mu$m | 39 |
| 5-10 $\mu$m | 31 |
| 10-20 $\mu$m | 26 |
| 20-50 $\mu$m | 4 |

Example 2

As same as in Example 1, microorganism cells were incubated, the microorganism suspension was prepared and viable cell count in the microorganism suspension was measured.

(Spray-granulation)

There was used a device where, in the fluidized layer granulator FLO-120 (manufactured by Freund Inc.), four-passage nozzle (that mentioned in the Japanese Patent No. 2,797,080) was integrated in the conventional two-passage spraying nozzle and then a lactic acid bacteria suspension was sprayed into the fluidized layer to prepare the powder containing the lactic acid bacteria.

The steps were roughly classified into two and, in the first step, a binder was blown upon an excipient as granulates using the two-passage nozzle to conduct the granulation. In the second step, a lactic acid bacteria suspension was spray-dried using the four-passage nozzle in the same state that the fluidized layer was kept at low temperature and, at that same time, it was mixed with the granulates obtained in the first step.

The operating condition of the machine used in this experiment was that the amount of supplied air was 70 m$^3$/minute and the amount of nozzle air was 40 NL/minute (value converted at 20°C).

Compositions of the excipient and the binder used for the granulation were as follows.

| | | |
|---|---|---|
| Excipient: | Corn starch | 40 kg |
| | Crystalline cellulose | 40 kg |
| | Lactose | 40 kg |
| Binder: | Corn starch | 1.8 kg |
| | Pure water | 60 L |

The first step will be illustrated in detail as follows. The above-mentioned excipient materials (120 kg) was weighed and placed in a hopper, temperature of supplying air was set at 100°C, dry air was sent therein to form a fluidized layer and the binder was sprayed using a two-passage nozzle at the liquid flow rate of 1.6 L/minute. The exhaust gas temperature at that time was 45-50°C and the temperature in the fluidized layer was 55-60°C. When spraying of the binder was finished, the fluidized layer was dried as it was for 20 minutes to give granulates containing low amount of water.

The second step will be now illustrated in detail as follows. After completion of the post-drying of the granulates, the microorganism suspension (350 L) prepared by the same manner as in Example 1 was sprayed at the liquid flow rate of 16 mL/minute using the four-passage nozzle under the state where the temperature of supplying air was lowered to 70°C. At that time, the exhaust gas temperature was 30-35°C and the temperature in the fluidized layer was 35-40°C. As a result of spray-drying at such a low temperature, lactic acid bacteria were pulverized and, when they were mixed in the fluidized layer at the same time, powder containing the lactic acid bacteria according to the present invention was obtained.

Comparative Example 2

There was prepared a mixed powder of lactic acid bacteria where an adjustment was done so as to contain the same amount of the lactic acid bacteria in the preparation.

The microorganism cell powder (2 g) prepared in Example 1 was diluted to an extent of ten-fold using corn starch to make 20 g, added to 1180 g of the granulates comprising the excipient and the binder prepared in the first step of Example 2 and mixed at 100 rpm for 3 minutes using a mixer of type V to prepare a mixed lactic acid bacteria powder.

Viable cell count contained in 1 g of the powder obtained in Example 2 or Comparative Example 2 was measured according to the item of (Measurement of viable cell count in the dried microorganism cell product) of Example 1. In Example 2, the viable cell count was $5.12 \times 10^8$ (CFU/g) and, in Comparative Example 2, it was $4.85 \times 10^8$ (CFU/g).

It has been therefore found that, in accordance with the present invention where spray-drying and fluidized layer granulation are combined, it is possible to simplify the manufacturing steps in which at least the same or even more viable cell rate can be still maintained as in the case of the conventional manufacturing method for the powder comprising spray-drying, granulation and mixing steps.

Example 3

To 500 g of distilled water, the following components were added followed by stirring to prepare a coating agent.

Composition of the coating agent:

| | |
|---|---|
| Eudragit L30D-55 | 500 g |
| Polyethylene glycol 6000 | 15 g |
| Tween 80 | 7.5 g |
| Talc | 45 g |

In the meanwhile, a microorganism suspension was prepared by the same manner as in Example 1.

Spray-drying was carried out using a spray-drier as shown in Fig. 2 where a four-passage nozzle (as mentioned in the Japanese Patent No. 2,797,080) was integrated. Thus, a microorganism suspension was supplied at the rate of 5 g/minute from one liquid passage in the four-passage nozzle comprising two liquid passages and two gas passages, a liquid coating agent was supplied at the rate of 20 g/minute from another liquid passage and compressed gas was supplied at the rate of 40 NL/minutes from the two liquid passages and the resulting spray-dried liquid droplets were dried by drying wind to manufacture a dried microorganism cell product which was partially coated according to the present invention.

Incidentally, inlet temperature of the drying wind was 100°C and the wind was supplied at the rate of 1.0 m³/ minute into a drying chamber of 0.9 mPa.

Each 10 mg of the conventional bifidobacterial powder and the partially-coated dried microorganism cell product according to this Example were taken and subjected to a palatability test whereupon the taste of the partially-coated dried microorganism cell product according to this Example was improved.

Example 4

The microorganism suspension (100 g) prepared in the same manner as in Example 1 and an enteric coating material having the following composition were mixed and the said mixture was spray-dried using a spray-drier as shown in Fig. 2 where a four-passage nozzle (as mentioned in the Japanese Patent No. 2,797,080) was integrated. Thus, a mixed solution was supplied from the two liquid passages at the rate of 5.6 g/minute while compressed gas was supplied from the two gas passages at the rate of 40 NL/minute and the resulting spray-dried droplets were dried by drying wind to manufacture an enteric-coated dried microorganism cell product according to the present invention.

Incidentally, inlet temperature of the drying wind was 80°C and it was supplied at the rate of 1.0 m³/minute into a drying chamber of 0.9 mPa.

Enteric coating material:

| | |
|---|---|
| AQOAT (manufactured by Shin-Etsu Chemical) | 30 g |
| Triethyl citrate | 8.4 g |
| Distilled water | 161.6 g |

(Acid resistance test of the enteric-coated dried microorganism cell product)

An acid resistance test was carried out using the first liquid (pH 1.2; 36-38°C) mentioned in "General Test Method - Disintegration Test Method" of the Japanese Pharmacopoeia. The first liquid was placed in a container of about 1000 mL which was able to keep warm and kept at 36-38°C. To this was added a dried microorganism cell product followed by well stirring and each 1 mL was sampled after 5, 15, 30 and 60 minute(s) from the addition of the dried microorganism cell product. The sampled specimen was successively diluted to appropriate concentrations using a diluent mentioned in Example 1 and measurement of viable cell count was carried out as same as in Example 1. Incidentally, the first liquid of the pharmacopoeia was an artificial gastric juice where 2.0 g of sodium chloride are added to and dissolved in 7 mL of hydrochloric acid and distilled water to make 1000 mL.

The same test was carried out for the conventional bifidobacterial powder as well.

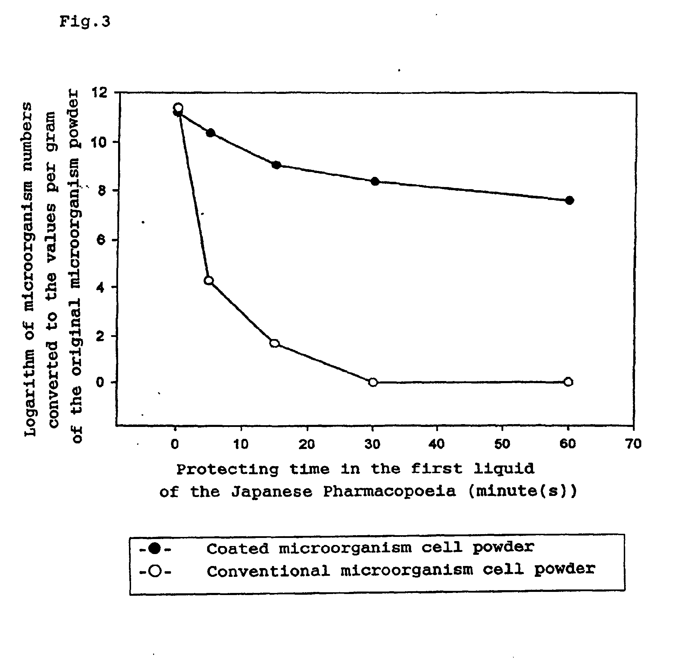

Fig. 3 shows the relation between the protected time of the first liquid and the viable cell count. In the conventional microorganism cell powder, viable cell count became 1/1000 or less after 5 minutes from pouring and, after 30 minutes and thereafter, no viable cell was noted while, in the coated microorganism cell powder according to the present invention, about 1% of viable cells were noted even after 60 minutes from pouring. Thus, as a result of conducting the above-mentioned coating, resistance of the dried microorganism cell product to acid was significantly improved.

Example 5

A microorganism suspension was prepared by the same manner as in Example 1 except that *Lactobacillus acidophilus* was used as the microorganism.

Viable cell count in the microorganism suspension was measured according to quantitative determination of lactomin mentioned under the item of "Lactomin" in the Standards for Pharmaceuticals Unlisted in the Japanese Pharmacopoeia. Thus, 1 mL of the microorganism suspension was taken and diluted with a diluent liquid prepared as mentioned below by a 10-fold dilution method so as to make the viable cell count 20-200 in 1 mL. This liquid (1 mL) was placed on a Petri dish and 20 mL of agar medium for the test of lactomin kept at 50°C were added thereto followed by quickly mixing to solidify. This was incubated at 37°C for 48 hours and viable cell count in the microorganism suspension was determined from the appeared colony numbers and the diluting ratio.

Here, the agar medium for the test of lactomin was prepared in such a manner that the following components were mixed and sterilized by heating at 121°C for 15 minutes using a high-pressure steam sterilizer.

The tomato juice used here was prepared in such a manner that the same amount of pure water was added to tomato juice and the mixture was boiled with occasional stirring, adjusted to pH 6.8 and filtered.

| | |
|---|---|
| Peptone made of casein | 20 g |
| yeast extract | 5 g |
| Meat extract | 15 g |
| Glucose | 20 g |
| L-Cysteine hydrochloride | 1 g |
| Polysolvate 80 | 3 g |
| Tomato juice | 200 mL |
| Agar | 20 g |
| Pure water | 800 mL |
| pH: 6.8 | |

The above diluent liquid was prepared in such a manner that the following components were mixed and sterilized by heating at 121°C for 15 minutes using a high-pressure steam sterilizer.

| | |
|---|---|
| Sodium monohydrogen phosphate | 6.0 g |
| Potassium dihydrogen phosphate | 4.5 g |
| Polysolvate 80 | 0.5 g |
| L-Cysteine hydrochloride | 0.5 g |
| Agar | 1.0 g |
| Pure water | 1000 mL |
| pH: 6.9 | |

The microorganism suspension prepared as above was spray-dried by the same manner as in Example 1.

The operation condition of the machine was as same as in Example 1 except that the inlet temperature was 60°C and the exhaust gas temperature was 45°C.

Viable cell count in the resulting dried microorganism cell product was measured according to quantitative determination of lactomin mentioned under the item of "Lactomin" in the Standards for Pharmaceuticals Unlisted in the Japanese Pharmacopoeia. Thus, 5 g of the dried microorganism cell product were taken by a diluent liquid which was previously warmed at 37°C to make the total volume 50 mL. This was well stirred and warmed at 37°C for 30 minutes to give an original specimen liquid. This was diluted with a diluent liquid by a 10-fold dilution method so as to make the viable cell count 20-200 in 1 mL. This liquid (1 mL) was placed on a Petri dish and 20 mL of agar medium for the test of lactomin kept at 50°C were added thereto followed by quickly mixing to solidify. This was subjected to an anaerobic culture at 37°C for 48 hours and viable cell count in the microorganism suspension was determined from the appeared colony numbers and the diluting ratio.

The viable cell count in the dried microorganism cell product was $7.24 \times 10^{11}$ (CFU/g) and the survival rate was 90%. Incidentally, the survival rate was determined by the same manner as in Example 1.

It has been therefore found that, when spray-drying in accordance with the present invention is used, survival rate of the microorganism cells is improved whereby a dried microorganism cell product containing a very high viable cell count is able to be prepared.

Industrial Applicability

As a result of using a spray-drying device equipped with a device for preparing fine particles by which sprayed liquid droplets in a single micron size can be formed, surface area per unit weight of the sprayed liquid droplets becomes bigger and contact with drying wind proceeds very efficiently. Accordingly, when drying is carried out by drying wind of the temperature which is in the same degree as that in the conventional spray-drying, time required for drying the microorganism cell liquid becomes shorter and extinction or damage of the microorganism cells can be suppressed as much as possible. Further, even when the temperature of drying wind is made lower than the temperature of drying wind in the conventional spray-drying, a sufficient drying is possible without deteriorating the yield or stability and, furthermore, since the temperature of drying wind is low, extinction or damage of the microorganism cells due to heat of drying wind can be suppressed as much as possible. Therefore, in accordance with the method for the manufacture of a dried microorganism cell product in accordance with the present invention, it is now possible to prepare a dried microorganism cell product containing much viable cell count.

Further, in the dried microorganism cell product containing the particles of a single micron size prepared by drying the sprayed liquid droplets having small particle size as such with drying wind, the pressure to be applied to the microorganism cells upon compressive molding, etc. is small and static electricity is hardly generated whereupon they are easily made into pharmaceutical or food in a preparation form such as tablets.

When a spray-drying device equipped with a four-passage nozzle comprising two liquid passages and two gas passages is used as a spray-drying device in the present invention, spraying in large amount is possible and manufacturing efficiency is improved because there are two liquid passages and, in addition, when two different solutions or suspensions are sprayed from different liquid passages at the same time, it is possible to manufacture a dried microorganism cell product wherein they are mixed.

Further, when a liquid masking agent or coating agent is sprayed from one of the liquid passages while a microorganism cell liquid is sprayed from another liquid passage, it is possible to manufacture a dried microorganism solid product which is masked or coated. Therefore, it is no longer necessary to carry out masking and coating separately whereby the manufacturing steps become simple. Similarly, when a capsule material is sprayed together with a microorganism cell liquid, it is also possible to manufacture microcapsules.

Furthermore, when a spray-drying device having a four-passage nozzle is used, particle size distribution becomes within a relatively narrow range and, accordingly, steps of particle size selection, sieving, etc. thereafter are easily carried out.

Still further, when a device which is **characterized in that** a step of conducting a fluidized layer granulation using powder and binder and a step of preparing a dried microorganism cell product of a single micron size by spray-drying are carried out in the same container in accordance with the present invention is used and there are continuously carried out granulation, spray-drying of microorganism cells and mixing in the said device, the manufacturing steps can be simplified and, at the same time, homogeneity of mixture of the dried microorganism cell product with the granulates is improved and classification hardly takes place as well.

**Claims**

1. A method for the manufacture of a dried microorganism cell product having a value of the particle size of 1-10 $\mu$m by rounding the first decimal factor, **characterized in that**, sprayed liquid droplets having a value of the particle size of 1-10 $\mu$m by rounding the first decimal factor are dried with drying wind.

2. The method for the manufacture of the dried microorganism cell product according to claim 1, wherein viable cell count in the dried microorganism cell product is from $1.0 \times 10^5$ to $2.0 \times 10^{12}$ CFU/g.

3. The method for the manufacture of the dried microorganism cell product according to claim 1 or 2, wherein the microorganism is bifido bacteria, lactobacillus, lactococci, sporogenous lactic acid bacteria, saccharification bacteria, yeasts, koji molds, actinomycetes, *Bacillus toyoi, B. licheniformis*, butyric acid bacteria, acetic acid bacteria or amino acid fermentation bacteria.

4. The method for the manufacture of the dried microorganism cell product according to any one of claims 1 to 3, wherein the microorganism is bifido bacteria.

5. The method for the manufacture of the dried microorganism cell product according to any one of claims 1 to 4, wherein the dried microorganism cell product is further coated or masked.

6. The method for the manufacture of the dried microorganism cell product according to any one of claims 1 to 5, wherein the sprayed liquid droplets having a value of the particle size of 1-10 $\mu$m by rounding the first decimal factor are formed by a four-passage nozzle.

7. The method for the manufacture of the dried microorganism cell product according to any one of claims 1 to 6, wherein the temperature of drying wind is 5-150°C at the inlet of a drying chamber.

8. The method for the manufacture of the dried microorganism cell product according to any one of claims 1 to 7, wherein a liquid coating agent or masking agent is sprayed together with a microorganism cell liquid to form sprayed liquid droplets and the said sprayed liquid droplets are dried with drying wind.

9. The method for the manufacture of the dried microorganism cell product according to claim 8, wherein the microorganism cell liquid is supplied from one liquid passage in a four-passage nozzle having two liquid passages and two gas passages, a liquid coating agent or masking agent is supplied from another liquid passage, compressed gas is supplied from the two gas passages and the resulting sprayed liquid droplets are dried by a drying wind.

10. The method according to any one of claims 1 to 9, **characterized in that**, a step where a fluidized layer granulation using powder and binder and a drying step where dried microorganism cell product having a value of the particle size of 1-10 $\mu$m by rounding the first decimal factor is obtained by spray-drying are carried out in the same container.

**Patentansprüche**

1. Verfahren zur Herstellung eines getrockneten Mikroorganismuszellproduktes mit einem Wert der Teilchengröße von 1 - 10 $\mu$m, wobei der erste Dezimalfaktor gerundet wird, **dadurch gekennzeichnet, dass** gesprühte Flüssigkeitstropfen mit einem Wert der Teilchengröße von 1 - 10 $\mu$m, wobei der erste Dezimalfaktor gerundet wird, mit Trockenluft getrocknet werden.

2. Verfahren zur Herstellung des getrockneten Mikroorganismuszellproduktes nach Anspruch 1, wobei die Lebendzellzahl in dem getrockneten Mikroorganismuszellprodukt 1,0 x 10$^5$ bis 2,0 x 10$^{12}$ KBE/g beträgt.

3. Verfahren zur Herstellung des getrockneten Mikroorganismuszellproduktes nach Anspruch 1 oder 2, wobei der Mikroorganismus Bifidobakterien, Lactobacillus, Lactococci, sporogene Milchsäurebakterien, Verzuckerungsbakterien, Hefen, Koji-Schimmelpilze, Actinomyzeten, *Bacillus toyoi, B. licheniformis*, Buttersäurebakterien, Essigsäurebakterien oder Aminosäure-Fermentationsbakterien ist.

4. Verfahren zur Herstellung des getrockneten Mikroorganismuszellproduktes nach einem der Ansprüche 1 bis 3, wobei der Mikroorganismus Bifidobakterien ist.

5. Verfahren zur Herstellung des getrockneten Mikroorganismuszellproduktes nach einem der Ansprüche 1 bis 4, wobei das getrocknete Mikroorganismuszellprodukt außerdem beschichtet oder maskiert ist.

6. Verfahren zur Herstellung des getrockneten Mikroorganismuszellproduktes nach einem der Ansprüche 1 bis 5, wobei die gesprühten Flüssigkeitstropfen mit einem Wert der Teilchengröße von 1 - 10 $\mu$m, wobei der erste Dezimalfaktor gerundet wird, durch eine Vierkanaldüse gebildet werden.

7. Verfahren zur Herstellung des getrockneten Mikroorganismuszellproduktes nach einem der Ansprüche 1 bis 6, wobei die Temperatur der Trockenluft am Einlass einer Trocknungskammer 5 - 150 °C beträgt.

**8.** Verfahren zur Herstellung des getrockneten Mikroorganismuszellproduktes nach einem der Ansprüche 1 bis 7, wobei ein flüssiges Beschichtungsmittel oder Maskierungsmittel unter Bildung gesprühter Flüssigkeitstropfen zusammen mit einer Mikroorganismuszellflüssigkeit gesprüht wird und die gesprühten Flüssigkeitstropfen mit Trockenluft getrocknet werden.

**9.** Verfahren zur Herstellung des getrockneten Mikroorganismuszellproduktes nach Anspruch 8, wobei die Mikroorganismuszellflüssigkeit aus einem Flüssigkeitskanal in einer Vierkanaldüse mit zwei Flüssigkeitskanälen und zwei Gaskanälen zugeführt wird, ein flüssiges Beschichtungsmittel oder Maskierungsmittel aus einem anderen Flüssigkeitskanal zugeführt wird, Druckgas aus den beiden Gaskanälen zugeführt wird und die resultierenden gesprühten Flüssigkeitstropfen mittels Trockenluft getrocknet werden.

**10.** Verfahren nach einem der Ansprüche 1 bis 9, **dadurch gekennzeichnet, dass** ein Fließbettgranulations-Schritt unter Verwendung eines Pulvers und eines Bindemittels und ein Trocknungsschritt, bei dem das getrocknete Mikroorganismuszellprodukt mit einem Wert der Teilchengröße von 1 - 10 μm, wobei der erste Dezimalfaktor gerundet wird, durch Sprühtrocknen erhalten wird, in demselben Behälter durchgeführt werden.

**Revendications**

**1.** Procédé de fabrication d'une cellule microbienne séchée possédant une valeur de taille de particules de 1 à 10 μm arrondie à la première décimale, **caractérisé en ce que** des gouttelettes de liquide pulvérisées possédant une valeur de taille de particules de 1 à 10 μm arrondie à la première décimale sont séchées avec un courant d'air de séchage.

**2.** Procédé de fabrication de la cellule microbienne séchée selon la revendication 1, dans lequel le nombre de cellules viables dans la cellule microbienne séchée est de $1,0 \times 10^5$ à $2,0 \times 10^{12}$ CFU/g.

**3.** Procédé de fabrication de la cellule microbienne séchée selon la revendication 1 ou 2, dans lequel le micro-organisme est une bactérie bifidus, un lactobacille, un lactococcus, une bactérie d'acide lactique à spores, une bactérie de saccharification, des levures, des moisissures de koji, des actinomycètes, Bacillus toyoi, B. licheniformis, une bactérie d'acide butyrique, une bactérie d'acide acétique ou une bactérie de fermentation d'un acide aminé.

**4.** Procédé de fabrication de la cellule microbienne séchée selon l'une quelconque des revendications 1 à 3, dans lequel le micro-organisme est une bactérie bifidus.

**5.** Procédé de fabrication de la cellule microbienne séchée selon l'une quelconque des revendications 1 à 4, dans lequel la cellule microbienne séchée est en outre enrobé ou marqué.

**6.** Procédé de fabrication de la cellule microbienne séchée selon l'une quelconque des revendications 1 à 5, dans lequel les gouttelettes de liquide pulvérisées possédant une valeur de taille de particules de 1 - 10 μm arrondie à la première décimale sont formées par une buse à quatre passages.

**7.** Procédé de fabrication de la cellule microbienne séchée selon l'une quelconque des revendications 1 à 6, dans lequel la température du courant d'air de séchage est de 5 à 150°C à l'entrée de la chambre de séchage.

**8.** Procédé de fabrication de la cellule microbienne séchée selon l'une quelconque des revendications 1 à 7, dans lequel un agent d'enduction ou un agent masquant liquide est pulvérisé avec le liquide de la cellule microbienne pour former des gouttelettes de liquide pulvérisées et lesdites gouttelettes de liquide pulvérisées sont séchées avec un courant d'air de séchage.

**9.** Procédé de fabrication de la cellule microbienne séchée selon la revendication 8, dans lequel le liquide de la cellule microbienne est alimentée depuis un passage de liquide dans une buse à quatre passages possédant deux passages de liquide et deux passages de gaz, un agent d'enduction ou un agent masquant liquide est alimenté depuis un autre passage de liquide, un gaz comprimé est alimenté depuis les deux passages de gaz et les gouttelettes de liquide pulvérisées résultantes sont séchées par un courant d'air de séchage.

**10.** Procédé selon l'une quelconque des revendications 1 à 9, **caractérisé en ce qu'**une étape, dans laquelle une granulation en couche fluidisée utilise une poudre et un liant, et une étape de séchage, dans laquelle une cellule microbienne séchée possédant une valeur de taille de particules de 1 à 10 $\mu$m arrondie à la première décimale est obtenue par séchage par pulvérisation, sont réalisées dans le même récipient.

Fig.1

Fig.2

Fig.3

Protecting time in the first liquid
of the Japanese Pharmacopoeia (minute(s))

-●- Coated microorganism cell powder
-O- Conventional microorganism cell powder

Fig.4

Exhaust fan

4 1

Liquid
-sending
device
4 3

Compressed
air

4 4

Liquid
-sending
device
4 5

Compressed
air

4 6

4 7

4 8

Aspirating filter

4 2

Heat exchanger

4 9

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0818529 A1
- WO 9800035 A
- WO 9902170 A

- JP 9048686 A
- JP 2797080 B

**Non-patent literature cited in the description**

- *Präventive Medizin,* vol. 177 (3-4), 251-256